# FASCICULE DE BREVET EUROPEEN

(11) **EP 2 309 249 B1**
(45) Date de publication et mention de la délivrance du brevet: **19.08.2020**
(21) Numéro de dépôt: 10186363.7
(22) Date de dépôt: 04.10.2010
(51) Int. Cl.: G01N 21/03, G01N 21/64, G01N 21/47, A61B 5/00, G02B 6/26, G02B 6/36

(54) **Dispositif et procédé pour l'excitation diffusée en imagerie**
Vorrichtung und Verfahren zur gestreuten Erregung in der Bildgebung
Device and method for diffused excitation in images

(30) Priorité: 08.10.2009 FR 0957029
(43) Date de publication de la demande: 13.04.2011
(73) Titulaire: Commissariat à l'Énergie Atomique et aux Énergies Alternatives, 75015 Paris (FR)
(72) Inventeur: Guyon, Laurent, 38100 Grenoble (FR); Boutet, Jérôme, 38640 Claix (FR); Debourdeau, Mathieu, 38830 Saint Pierre d'Allevard (FR)
(74) Mandataire: Ilgart, Jean-Christophe

(56) Documents cités:
- WO-A1-96/20638
- US-A- 6 138 046
- US-A1- 2005 226 548
- US-A1- 2009 046 291
- US-A1- 2009 153 850

## Description

### DOMAINE TECHNIQUE ET ART ANTÉRIEUR

L'invention concerne le domaine de l'imagerie optique diffuse, appliquée au domaine médical et notamment l'imagerie de fluorescence in vivo et ex-vivo.

Ces techniques permettent de mettre en œuvre des systèmes de diagnostic non invasifs grâce à l'utilisation de rayonnements non-ionisants, faciles d'utilisation et peu coûteux.

Une application de d'imagerie optique diffuse est l'imagerie de fluorescence, selon laquelle un marqueur fluorescent ou fluorophore (substance chimique d'une molécule capable d'émettre de la lumière de fluorescence après excitation) est injecté à un sujet à examiner et se fixe sur certaines molécules spécifiques, par exemple des tumeurs cancéreuses. La zone d'intérêt est ensuite éclairée à la longueur d'onde d'excitation optimale du fluorophore. Puis un signal de fluorescence est détecté.

Il existe également une technique d'imagerie optique diffuse, sans injection de marqueur fluorescent. On cherche dans ce cas à analyser un signal à la même longueur d'onde que la longueur d'onde d'excitation.

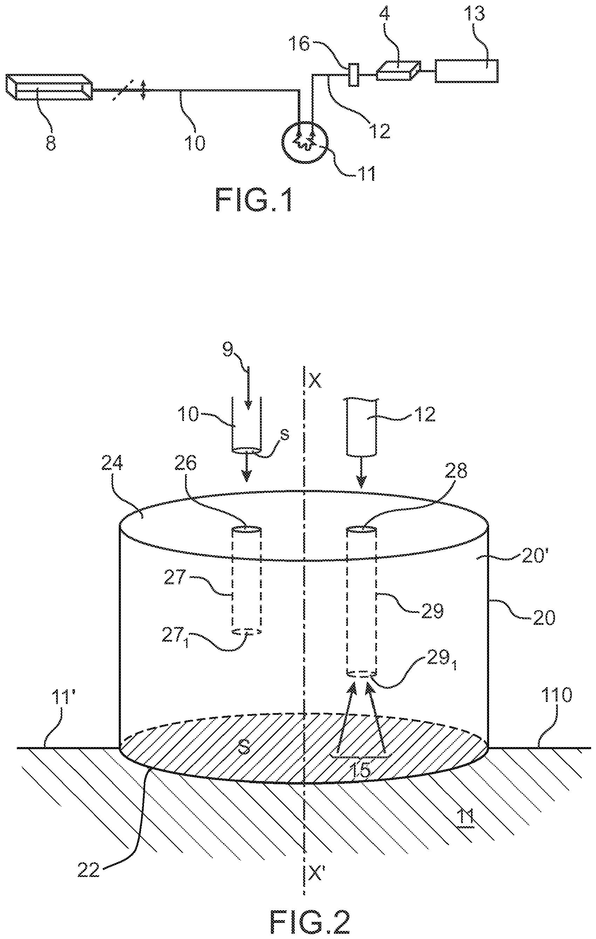

Un exemple de dispositif pour mettre en œuvre l'une de ces techniques de tomographie de fluorescence est représenté en figure 1.

Un laser 8 d'excitation, par exemple en Saphir-titane délivre un train d'impulsion à un certain taux de répétition, par exemple 80 Mhz, avec une puissance moyenne de quelques centaines de milliwatts en sortie de fibre l'excitation 10. Ce laser peut être accordable en longueur d'onde, pour exciter différents types de fluorophores Le laser, injecté dans la fibre optique 10 d'excitation, va permettre de sonder l'échantillon 11, un milieu diffusant dans lequel un marqueur fluorescent est inclus, ou pas, suivant la nature de la technique utilisée.

Si un marqueur fluorescent est présent dans le milieu, la fluorescence qu'il émet est collectée par une deuxième fibre optique 12 - de détection - et le signal de fluorescence filtré (la référence 16 désigne un filtre) est mesuré à l'aide d'un détecteur, par exemple un tube photomultiplicateur 4 raccordé à des moyens 13 permettant de mesurer le signal de fluorescence.

S'il n'y a pas de marqueur fluorescent, on est dans le cas d'un examen de diffusion optique, et c'est le signal diffusé qui est collecté par la deuxième fibre optique 12, le filtre 16 étant alors adapté à la longueur d'onde d'excitation.

Quelle que soit la technique utilisée, afin d'avoir une meilleure sensibilité, on peut être tenté d'injecter beaucoup d'énergie laser dans le milieu examiné. Mais il se pose un problème d'endommagement de ce milieu, à partir d'une certaine densité d'énergie. C'est notamment le cas lorsque l'on travaille sur un tissu, et notamment sur un tissu vivant, par exemple un tissu d'un organe humain (sein, prostate, cerveau, testicules, bras, carotide, thyroïde...)

On peut essayer d'élargir géométriquement l'impulsion. Mais, outre le fait que les tissus vont évacuer moins facilement la chaleur, la résolution de l'image va être dégradée, et ce dès que la fibre 10 a un diamètre de l'ordre de 1mm.

Un autre problème est celui de la sensibilité: on souhaite collecter au mieux les photons qui proviennent du milieu 11 examiné. On cherche en effet à minimiser les pertes, notamment aux interfaces.

Un autre problème est celui des effets de bord qui gênent la reconstruction, ces derniers étant difficilement modélisés avec précision. On constate donc que les frontières du milieu sont une cause d'erreur dans la reconstruction des propriétés du milieu.

### EXPOSÉ DE L'INVENTION

L'invention concerne d'abord un dispositif d'imagerie optique diffuse d'un milieu selon la revendication 1.

Dans la suite, les expressions matrice ou masse ou matrice diffusante ou masse diffusante sont utilisées indifféremment.

Lors d'une utilisation, la matrice est mise en contact avec l'échantillon ou l'organe examiné.

Un tel dispositif peut comporter :
- au moins deux logements, chaque logement étant apte à recevoir l'extrémité d'une fibre optique,
- ou au moins une fibre dont une extrémité est disposée de manière permanente dans le matériau diffusant et au moins un logement, apte à recevoir l'extrémité d'une fibre optique,
- ou au moins deux fibres, chacune ayant une extrémité disposée de manière permanente dans le matériau diffusant.

Plus généralement, un nombre quelconque de logements et/ou d'extrémités de fibres peuvent être prévus dans la même matrice, le ou les logements étant destinés à recevoir une ou plusieurs fibres (d'excitation et/ou de collection).

En particulier, une fibre de collection du signal, qu'il s'agisse d'un signal de fluorescence ou de diffusion, peut être insérée, de manière permanente ou dans un logement, à l'intérieur de la matière diffusante de la masse ou de la matrice. On constate alors une efficacité améliorée de collection du signal, par rapport au cas où cette fibre est située en dehors du dispositif de couplage selon l'invention.

L'utilisation d'une matrice a pour effet d'éloigner la frontière du milieu, par rapport aux extrémités de la ou des fibres d'excitation et/ou de la ou des fibres de collection, ce qui limite la perturbation liée aux effets de bords. Dans le cas d'une matrice épaisse, dont l'épaisseur (suivant une direction sensiblement perpendiculaire à la surface d'appui), dépasse quelques cm (par exemple est supérieure à 3 cm ou 5 cm), l'influence des bords devient négligeable; on se rapproche alors des conditions d'un milieu infini, ce qui, ensuite, facilite et améliore la reconstruction des propriétés optiques du milieu.

Selon l'invention:
- l'un des logements d'une fibre optique de collection a un fond situé à une distance (h) de la surface d'appui inférieure à la distance (H) à laquelle est situé le fond d'au moins un logement et/ou l'extrémité d'une fibre optique d'excitation,
- et/ou l'une des fibres optiques de collection a une extrémité disposée de manière permanente dans la masse diffusante, à une distance (h) de la surface d'appui inférieure à la distance (H) à laquelle est situé le fond d'au moins un logement et/ou l'extrémité d'une fibre optique d'excitation.

Ainsi, on peut positionner l'extrémité d'une ou plusieurs fibre d'excitation plus loin de l'interface avec le milieu étudié que l'extrémité d'une ou plusieurs fibre de collection, afin de maximiser l'énergie collectée. Selon un mode préféré, la hauteur h sera choisie nulle pour au moins une fibre de collection, de façon à ce qu'au moins une fibre de collection soit en contact avec le milieu, ou au plus près de celui-ci, cela afin de maximiser l'énergie collectée.

Si une fibre, qui transmet un signal d'excitation ou incident, est insérée dans un logement de la matrice ou de la masse en matériau diffusant, ou a son extrémité contenue de manière permanente dans cette masse, cette dernière réalise une diffusion du rayonnement d'excitation. Cette diffusion est similaire à celle du milieu examiné, si le milieu diffusant a des propriétés optiques proches de celles du milieu étudié. On entend par proche un écart relatif inférieur à 30%, préférentiellement 20%, pour au moins un des paramètres considérés : coefficient d'absorption ou coefficient de diffusion réduit.

Dans ce cas l'interface et le milieu étudié peuvent être considérés comme un seul milieu diffusant. En outre ceci simplifie les calculs lors de l'étape de reconstruction.

De préférence le milieu diffusant a donc des coefficients d'absorption et de diffusion réduits et d'absorption µₐ et µ_{s'} proches de ceux des tissus du milieu examiné.

Le matériau diffusant a, selon l'invention, un coefficient de diffusion réduit µ_{s'} supérieur à 0,1 cm⁻¹ et inférieur à 700 cm⁻¹, et de préférence compris entre 1 et 50 cm⁻¹, et encore de préférence compris entre 5 et 20 cm⁻¹.

Il peut avoir un coefficient d'absorption µₐ supérieur à 0,01 cm⁻¹ et inférieur à 10 cm⁻¹. De préférence, ce coefficient sera inférieur au coefficient de diffusion réduit µ_{s'}. On préférera des valeurs comprises entre 0,01 cm⁻¹ et 1 cm⁻¹.

Une couche absorbante peut recouvrir partiellement ou au moins au moins partiellement la masse en matériau diffusant. Il peut s'agir d'une couche de peinture noire, ou d'un métal anodisé recouvrant partiellement ou au moins partiellement la matrice. De préférence, la surface de contact avec le milieu est exempte de cette couche.

La tache du faisceau d'excitation, sur la surface d'appui, destinée à être en contact avec l'objet à examiner, a de préférence une surface Sₜ comprise entre 1 mm² et 1 cm² ou quelques cm², par exemple 5 cm², ou de l'ordre d'une dizaine de cm², ou de quelques dizaines de cm², par exemple comprise entre 10 cm² et 20 cm² ou 50 cm².

Selon encore un autre mode particulier de réalisation de l'invention, la surface d'appui comporte en outre une ou plusieurs protubérances de forme sensiblement arrondie et non anguleuse, prolongeant la surface d'appui, à appliquer contre l'objet à examiner.

Un tel dispositif peut avantageusement comporter au moins un logement, ou l'extrémité d'au moins une fibre, dont le fond est situé sensiblement dans, ou au niveau de, une telle protubérance.

Selon l'invention on fait provenir l'excitation ou le faisceau incident d'une source, généralement ponctuelle, située à l'extérieur ou à l'intérieur de la masse diffusante, mais on éclaire le tissu largement du fait de la diffusion du signal d'excitation dans la masse diffusante.

Le point source peut avoir un diamètre inférieur à 3mm, de préférence inférieur à 500 µm, tandis que le tissu est éclairé par une tache d'un diamètre d'au moins 1 mm ou 5 mm ou 10 mm et de préférence inférieur à 20 mm ou à 5 cm. Ce point source peut être situé dans la matrice, ce cas correspondant notamment à une fibre optique dont une extrémité est incluse dans la masse diffusante, cette extrémité pouvant alors être assimilée au point source. Il s'agit notamment du cas ou la fibre d'excitation a son extrémité insérée dans une ouverture prévue dans la masse diffusante ou du cas ou la fibre d'excitation a son extrémité fixée de manière permanente dans la masse diffusante, par exemple par moulage. Mais la source de lumière peut être, dans certains cas, située à distance de la masse diffusante.

Une portion d'extrémité d'une des fibres peut n'être constituée que du cœur si la matrice joue le rôle de gaine.

La source de rayonnement peut être un laser. La source de rayonnement peut être une source en impulsions, ou continue en temps ou modulée en amplitude. La technique d'imagerie optique diffuse mise en œuvre peut être de type résolue en temps, ou bien de tout autre type. Cette technique peut être appliquée à l'imagerie optique diffuse de fluorescence, par exemple en tomographie de fluorescence, ou à la détermination de propriétés optiques de milieux diffusants.

L'invention a en outre pour objet un procédé d'imagerie optique diffuse d'une partie d'un milieu, dans lequel on utilise au moins un dispositif selon l'une des revendications 1 à 7, et dans lequel :
- on applique contre ce milieu le dispositif de couplage,
- on réalise un éclairement ou une excitation optique du milieu à l'aide des moyens formant source de rayonnement pour former un rayonnement incident sur le milieu, puis pour introduire ce rayonnement incident dans le milieu,
- on prélève un signal optique en provenance du milieu, à l'aide d'au moins une fibre optique de collection.

De préférence, on applique un fluide entre la surface d'appui du milieu diffusant du dispositif de couplage et la surface du milieu à examiner.

Avec ou sans fluide de couplage on a pu constater, de façon surprenante, que, dans le cas où le milieu à examiner présente une certaine souplesse, par exemple dans le cas de tissus vivants, le fait d'exercer une pression sur le dispositif de couplage positionné contre le milieu à examiner permet d'accroître le signal de réception. Lors de la mise en œuvre d'un dispositif selon l'invention, comportant au moins une fibre d'excitation et/ou au moins une fibre de collection, on pourra donc exercer avantageusement une pression pour appliquer le dispositif de couplage contre le milieu étudié, de manière à faire refluer un fluide contenu par ce milieu hors d'une partie de ce milieu, vers une zone située à la périphérie du dispositif de couplage .

### BRÈVE DESCRIPTION DES DESSINS

- La figure 1 représente un dispositif pour mettre en œuvre un procédé selon l'art antérieur,
- La figure 2 est un exemple d'un mode de réalisation de l'invention, avec une matrice de diffusion cylindrique,
- La figure 3 est un exemple d'un autre mode de réalisation de l'invention, avec une matrice de diffusion sensiblement hémisphérique,
- La figure 4 est encore un exemple d'un autre mode de réalisation de l'invention, avec une matrice de diffusion pour chaque fibre,
- Les figures 5A et 5B sont un exemple d'un autre mode de réalisation de l'invention, avec deux fibres d'excitation et une fibre de collection de signal ou deux fibres de collection de signal et une fibre d'excitation,
- Les figures 6A - 6D sont divers exemples d'un mode comparatif, avec un ou plusieurs logements apte à recevoir une ou plusieurs fibres d'excitation (figures 6A et 6C) ou de collection du signal de fluorescence (figures 6B et 6D),
- La figure 7 représente une utilisation d'un dispositif selon l'invention, dans laquelle une pression est appliquée sur ce dispositif lorsqu'il est en contact avec la surface d'un milieu à analyser,
- La figure 8A est un exemple d'un autre mode de réalisation de l'invention, avec une matrice munie d'une ou plusieurs excroissances ;
- Les figures 8B - 8D sont des exemples comparatifs.
- Les figures 9A - 9C représentent une matrice d'un dispositif selon l'invention, une tâche faite par un éclairement sur la surface d'appui de cette matrice et un profil d'intensité le long d'une section de cette tache.
- Les figures 10A et 10B sont des exemples d'encore un autre mode de réalisation de l'invention, avec des fibres dont les extrémités sont disposées de manière permanente dans le matériau de la matrice.

### EXPOSÉ DÉTAILLÉ DE MODES DE RÉALISATION PARTICULIERS

Un premier exemple d'un mode de réalisation de l'invention est donné en figure 2.

On retrouve sur cette figure les fibres 10, 12 respectivement pour amener, dans un milieu diffusant à étudier 11, un faisceau 9 d'excitation (ou, plus généralement, un faisceau incident) et pour en prélever un signal de fluorescence ou de diffusion en provenance de ce même milieu.

Dans cet exemple les extrémités des deux fibres 10, 12 sont introduites dans un élément d'interface 20 en un matériau diffusant. On utilise par la suite l'expression « matrice », mais l'un des termes « interface » ou « masse » peut être utilisé indifféremment à la place. Cette matrice peut être en un matériau solide Mais elle peut aussi être en un matériau mou, visqueux ou liquide, c'est alors par exemple de l'intralipide.

Cette matrice a ici une forme sensiblement cylindrique, avec deux faces 22, 24 perpendiculaires à l'axe XX' de révolution du cylindre. L'une de ces faces (la face 24 sur la figure 2) comporte deux ouvertures 26, 28 pour deux cavités ou logements 27, 29. Ces logements sont destinés à recevoir les extrémités des deux fibres d'excitation 10, et de collection 12 lorsque celles ci sont introduites par les ouvertures 26, 28. Autrement dit, chacune des ouvertures 26, 28, qui est de préférence sensiblement circulaire, est prolongée dans la masse de matériau diffusant de la matrice 20 par une paroi qui définit une cavité 27, 29 qui est de préférence sensiblement cylindrique, adaptée à la fibre. Chaque cavité a une extrémité fermée 27₁, 29₁ tournée vers la surface 22 destinée à être en contact avec le milieu 11 à étudier.

Chaque extrémité fermée 27₁, 29₁ est aussi la partie du logement 27, 29 la plus proche de cette surface 22. Par l'extrémité 27₁, le faisceau d'excitation 9 sort de la fibre d'excitation 10 et pénètre dans la matrice; par l'extrémité 29₁ le faisceau de diffusion 9 sort de la matrice et est récupéré par la fibre 12 de collection. Chaque cavité ainsi définie dans la matrice est adaptée à recevoir au moins une fibre. Le diamètre de chaque ouverture 26, 28 est donc sensiblement celui de la fibre, ou des fibres, qu'elle est destinée à recevoir. Un liquide de couplage peut être placé au fond de l'une et/ou de l'autre des cavités; l'indice de réfraction de ce liquide est préférentiellement proche de celui du matériau de la matrice.

De façon avantageuse, pour améliorer le couplage optique entre la matrice et le milieu étudié 11, et donc pour faciliter la modélisation de la propagation des rayons lumineux dans le système diffusant composé de la matrice et du milieu 11, les propriétés optiques de diffusion et, en outre, éventuellement d'absorption, du matériau de la matrice peuvent être choisies proches de celles du milieu à étudier.

Ainsi, le coefficient de diffusion réduit µ'ₛ du matériau constituant la matrice est supérieur à 0,1 cm⁻¹ et inférieur à 700 cm⁻¹ et de préférence compris entre 1 et 50 cm⁻¹, et encore de préférence compris entre 5 et 20 cm⁻¹, ces valeurs étant bien adaptées à des longueurs d'onde d'excitation ou de fluorescence dans le rouge ou l'infra rouge.

Le coefficient d'absorption µₐ du matériau constituant la matrice peut varier entre 0 cm⁻¹ et 10 cm⁻¹ et de préférence comprise entre 0,01 cm⁻¹ et 1 cm⁻, qui sont, là encore, bien adaptées à des longueurs d'onde d'excitation ou de fluorescence dans le rouge ou l'infra rouge.

Un faible coefficient d'absorption µₐ permet de ne pas perdre trop de signal et de ne pas donner lieu à un échauffement trop important de la matrice (toute absorption entraîne une élévation de température). De préférence on réalise une matrice 20 ayant le même coefficient d'absorption µₐ que la matière 11 examinée afin de constituer un milieu le plus homogène possible, cela afin de diminuer l'influence de l'interface et de faciliter la reconstruction. Ainsi, dans certains cas, on optera pour un faible coefficient d'absorption, tandis que pour d'autres applications, on préférera un coefficient d'absorption proche de celui du milieu considéré.

Une composition de type résine-encre-dioxyde de titane est un matériau adapté On peut aussi utiliser un polymère diffusant ou un polymère transparent auquel on ajoute des particules diffusantes, ou du cryogel. Le cryogel est un composé d'alcool polyvinyllique, souvent désigné par l'acronyme PVA (Polyvinyl Alcohol), dont la consistance peut-être rendue plus ou moins visqueuse, voire solide, en lui faisant subir des cycles de congélation - décongélation. En variante on peut également utiliser des matériaux visqueux ou solide, comprenant du gel d'agarose ou de la gelatine animale. De préférence, l'indice de réfraction du matériau sera choisi voisin de celui du milieu étudié.

Le rayonnement d'excitation 9 utilisé peut notamment être dans l'infra rouge, il a par exemple une ou des longueurs d'onde qui peuvent être comprises entre 400 nm et 1300 nm, de préférence entre 600 nm et 950 nm. Le signal collecté 15 a soit une longueur d'onde plus grande que la ou les longueurs d'onde du faisceau d'excitation (dans le cas d'un signal de fluorescence provenant d'un marqueur exogène), soit une longueur d'onde sensiblement identique ou voisine à la ou aux longueurs d'onde du faisceau d'excitation (cas d'un signal de diffusion).

La surface 22, ou une face d'appui, permet au dispositif d'être appliquée contre la surface 11' du milieu 11 à examiner. En général, cette surface 22 est plane, ce qui lui permet d'être appliquée contre une surface qui est elle-même au moins en partie plane.

Mais elle peut aussi être courbe ou présenter une courbure; on verra ci-dessous que d'autres surfaces sont adaptées, en particulier pour l'examen de tissus mous ou souples, avec des protubérances aux formes arrondies ou encore avec au moins un rayon de courbure non nul dans un plan perpendiculaire à la surface du milieu à examiner.

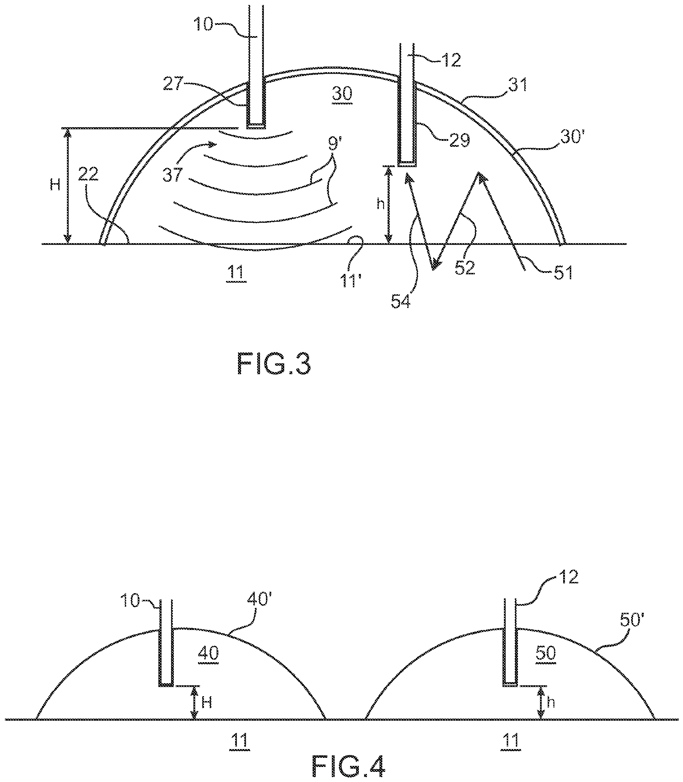

La matrice 20 peut être de forme cylindrique, comme sur la figure 2 : elle a alors une surface extérieure 20' à symétrie de révolution autour d'un axe XX', sensiblement perpendiculaire à la surface 22, mais ce n'est qu'un exemple et une telle symétrie n'est pas nécessairement présente. La forme de la matrice n'est pas limitée à cet exemple, et sa surface non destinée à être en contact avec le milieu ou le tissu à examiner peut être de forme cubique, ou parallélépipédique, ou en demi-sphère, comme la surface 30' de la matrice 30 de la figure 3, ou selon un autre exemple, comporter une surface 30', 40', 50' (voir figures 4-8D) courbée et une surface plane 22, par exemple en forme de demi ellipsoïde. Dans tous les cas elle peut posséder au moins une ou au moins deux ouvertures 26, 28, chacune prolongée par une cavité 27, 29 de forme sensiblement cylindrique, adaptée à recevoir l'une des fibres.

Quel que soit le mode de réalisation envisagé, la matrice solide a une surface extérieure 20', 30', 40', 50' destinée, en cours d'utilisation, à ne pas être en contact avec la surface du milieu étudié. Cette surface peut être recouverte en partie d'une couche absorbante 31, 41, 51 formée par exemple d'une couche de peinture sombre, ou d'une couche en métal anodisé, mais pas dans les zones d'ouverture 26, 28 qui permettent à la ou aux fibres 10, 12 d'être positionnées dans la matrice, et pas sur la surface 22 à mettre en contact avec le milieu à analyser.

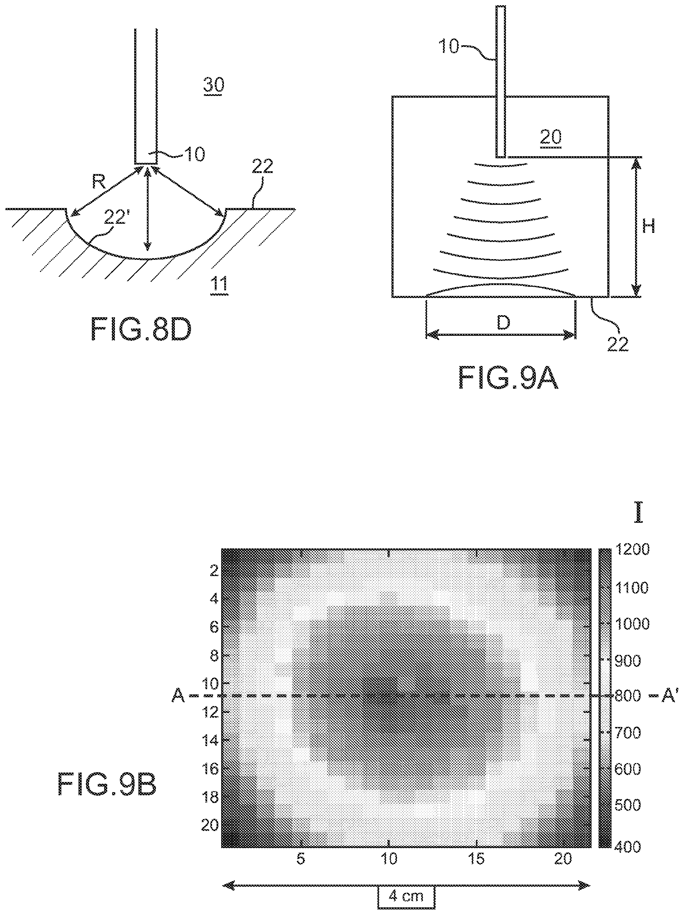

D'une manière générale, si une fibre d'excitation 10 est introduite dans un dispositif de couplage selon l'invention, la source apparaît comme ponctuelle dans le milieu diffusant de la matrice. Mais il y a un effet de diffusion du rayonnement d'excitation dans cette matrice, avant que celui-ci n'atteigne la surface 11' du milieu 11 étudié. Ainsi sur la figure 3 on a représenté la diffusion du faisceau d'excitation, en provenance de la fibre 10, sous la forme d'une onde 9' qui diffuse à partir de l'extrémité de cette fibre optique avant d'atteindre la surface 11'. Les figures 9A - 9C, qui seront décrites plus loin de manière plus détaillée, donnent un exemple de l'effet d'élargissement qu'un dispositif selon l'invention permet d'obtenir.

L'impact du faisceau incident sur ce milieu n'est donc pas ponctuel, mais réparti sur une surface plus importante que si la fibre était utilisée seule, sans dispositif de couplage selon l'invention. La densité surfacique de signal est donc plus faible que si l'excitation était dirigée, avec une même intensité de signal d'excitation, vers le milieu à examiner sans dispositif selon l'invention.

Les dommages dans le milieu à examiner sont donc minimisés, voire évités. Cependant, la source est considérée comme restant ponctuelle et le faisceau Laser ou la fibre optique peuvent être modélisés comme étant des sources ponctuelles. La matrice diffusante peut, en outre, être prise en compte dans le modèle utilisé pour la reconstruction, le milieu diffusant incorporant la matrice diffusante.

La profondeur de chaque logement 27, 29 est adaptée à la fonction de la fibre qu'elle doit recevoir.

En particulier il est en effet préférable que la distance entre l'extrémité de la fibre 10 d'excitation et la surface 11' de la matière à analyser soit supérieure au libre parcours moyen de diffusion isotrope, ce dernier étant l'inverse du coefficient de diffusion réduit (= 1/µ_{s'}), dans le milieu diffusant, des photons d'excitation.

En général, quel que soit le mode de réalisation, la surface 22, destinée à être en contact avec la zone qui délimite le milieu diffusant, est plus proche de l'extrémité fermée 29₁ de la cavité 29, destinée à recevoir la fibre 12 de collection du signal, que de l'extrémité fermée 27₁ de la cavité 27 destinée à recevoir la fibre 10 d'excitation. Selon l'invention, la hauteur H qui sépare la surface 22 de l'extrémité 27₁ de la cavité 27, extrémité qui est la plus proche de cette même surface, est supérieure à la hauteur h qui sépare cette surface 22 de l'extrémité 29₁ de la cavité 29 la plus proche de la surface 22. Par exemple, H est compris entre 0 et 5cm, préférentiellement entre 1 mm et 10 mm, tandis que h est compris entre 0 et 5cm, préférentiellement entre 0 et 5mm, et encore préférentiellement voisin de 0.

L'efficacité de collection du signal est d'ailleurs accrue lorsque l'extrémité de la fibre 12 de collection est proche de l'interface 22. En effet certains photons suivant le trajet référencé 51, 52, 54 (représentés en figure 3) qui proviennent du milieu étudié et qui ne sont pas dirigés directement vers l'extrémité de la fibre 12 vont alors être diffusés dans la masse de la matrice 20 et/ou du milieu étudié 11 et vont donc être ramenés vers l'extrémité de la fibre 12 ; ils vont subir des réflexions ou vont être diffusés dans cette masse de la matrice 20 ou du milieu 11 (voir le trajet 51-52-54 sur la figure 3) et une partie d'entre eux sera captée par la fibre 12 ; ces photons 51 seraient perdus pour le signal en l'absence de matériau diffusant disposé entre le milieu 11 et l'extrémité 29₁ de la fibre de collection

De préférence encore, la tache 37 du faisceau d'excitation, telle que représentée sur la figure 3, sur la face d'appui 22, destinée à être en contact avec l'objet à examiner, a une surface Sₜ de l'ordre de quelques mm² à quelques cm² voire quelques dizaines de cm², par exemple comprise entre 10 mm² et 10 cm². On peut considérer que le diamètre de cette tache du faisceau d'excitation sur la face d'appui 22 est la largeur à mi hauteur de l'intensité maximum de la tache de diffusion. Si cette tache 27 est sensiblement circulaire, son diamètre, au niveau de la face d'appui 22, est par exemple compris entre 500 µm et 1 cm ou quelques cm. En sortie de la fibre 10, le diamètre s de la tache lumineuse est de quelques centaines de µm, par exemple compris entre quelques µm et quelques centaines de µm, voire 1 mm. Ceci est aussi valable pour les autres réalisations de l'invention, dès lors qu'une fibre d'excitation est insérée dans le dispositif de couplage selon l'invention.

En notant s la surface de l'extrémité de la fibre et S la surface 22 de la matrice 20 diffusante, la prise en compte de l'énergie maximale autorisée sera d'autant plus faible que S sera grand, et la résolution spatiale de la reconstruction dépendra notamment de s. Les figures 9A - 9C permettent d'expliquer l'élargissement d'un faisceau pénétrant dans le milieu 11 examiné en utilisant un dispositif selon l'invention (figure 9A), du type de celui décrit ci-dessus en liaison avec la figure 2, mais dans lequel une seule fibre, à savoir la fibre d'excitation ou de faisceau incident 10, pénètre. Le rayonnement qui est envoyé dans cette fibre forme, sur la surface 22 de sortie, destinée à être en contact avec le milieu étudié, une tache de diamètre sensiblement égal à D. Dans ce dispositif, la fibre a un diamètre d'environ 60 µm, et son extrémité est distante de la surface 22 d'une distance égale à environ 6 cm. Les coefficients µₐ et µ'ₛ du matériau de la matrice sont respectivement de 0,03 cm⁻¹ et 9 cm⁻¹.

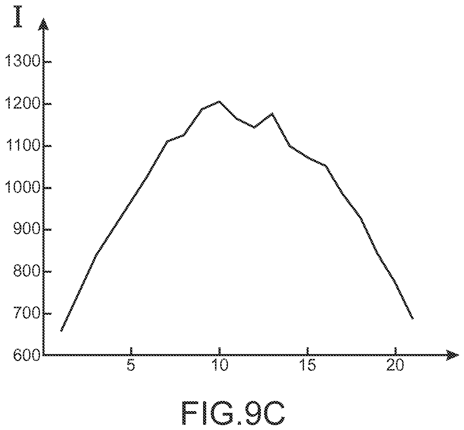

La figure 9B représente une image de cette tache dans le plan de la surface 22, l'intensité étant affichée sur l'échelle de droite, entre 400 et 1200 en unités arbitraires. La figure 9C représente le profil d'intensité le long d'un axe AA' qui se passe dans le plan de cette tache, sensiblement par la zone d'intensité maximum. On voit que l'on peut définir, à l'aide de cette courbe, une largeur à mi hauteur, qui est ici d'environ 4 cm. On peut définir le diamètre du faisceau incident, au niveau de la surface 22, par cette largeur à mi-hauteur. Sur cet exemple, on comprend également que le dispositif selon l'invention permet effectivement d'élargir considérablement la surface d'éclairement du faisceau incident, puisque celui-ci passe, dans cet exemple, d'un diamètre de 60 µm (la sortie de la fibre 10) à un diamètre de plusieurs centimètres. Naturellement, on observerait le même effet d'élargissement du faisceau d'excitation si la source était une fibre, ou une source Laser, située à l'extérieur de la matrice, mais produisant un faisceau, par exemple ponctuel, traversant la matrice 30 avant d'atteindre le milieu diffusant 11. On retrouverait alors un des avantages de cette matrice diffusante, qui est l'élargissement du faisceau incident, permettant d'utiliser une source lumineuse de forte intensité sans pour autant endommager le milieu examiné. Cet effet technique provient de l'atténuation de la densité surfacique du signal résultant de l'élargissement du signal d'excitation, cet élargissement se produisant sous l'effet de la diffusion de ce signal d'excitation dans la matrice 30, avant d'atteindre le milieu 11.

Dans les exemples ci-dessus, la matrice comporte deux ouvertures et deux logements, un pour chacune des fibres 10, 12.

En variante, il est également possible d'utiliser une matrice pour chaque fibre, comme en figure 4: pour une fibre d'excitation 10 et une fibre 12 de collection, on utilise alors deux matrices 40, 50 selon l'invention, positionnées de manière voulue sur la surface de l'objet examiné. Suivant la nature (excitation ou collection) de la fibre introduite dans chaque matrice, la profondeur de pénétration de la fibre dans cette matrice peut être plus ou moins importante: la fibre 10 d'excitation peut pénétrer moins profondément dans une matrice 40 (profondeur H sur la figure 4), que la fibre 12 dans la matrice 50 (profondeur h<H sur la figure 4) afin de bénéficier des effets décrits ci-dessus, notamment pour capter les rayons provenant du milieu étudié. Si plusieurs fibres sont utilisées pour amener chacune un faisceau d'excitation, on peut juxtaposer plusieurs matrices, une pour chaque faisceau, et une ou plusieurs matrices pour la collection du signal.

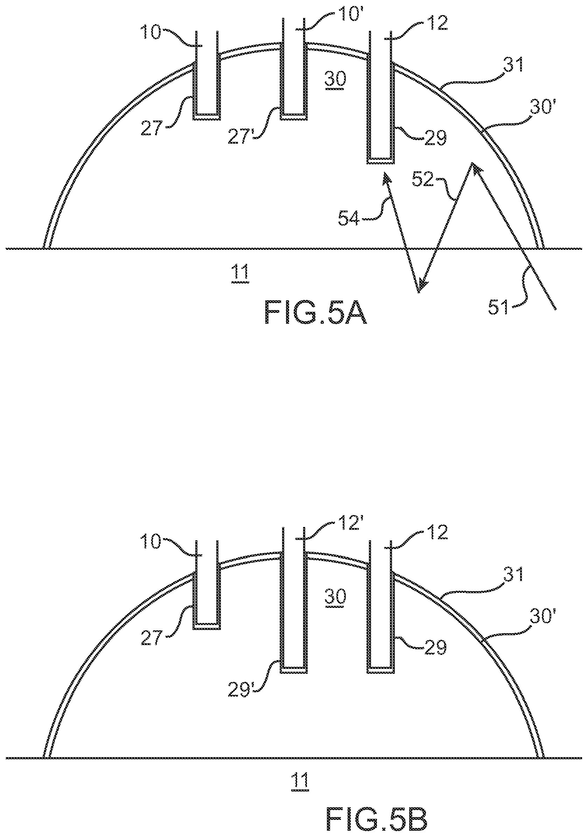

En variante encore, il est également possible d'utiliser une matrice pour plusieurs fibres, comme en figure 5A: par exemple plusieurs fibres d'excitation 10, 10' et une fibre 12 de collection, sont positionnées dans la même matrice 30. Mais on peut aussi avoir une seule fibre d'excitation 10 et une pluralité de fibres de collection 12, 12' (figure 5B), mais, de façon préférée, une pluralité de fibres d'excitation 10, 10', par exemple de 1 à 100 fibres d'excitation, et une pluralité de fibres de collection, par exemple de 1 à 100 fibres de collection.

Là encore la profondeur de chaque logement est de préférence adaptée à la nature de la fibre à recevoir.

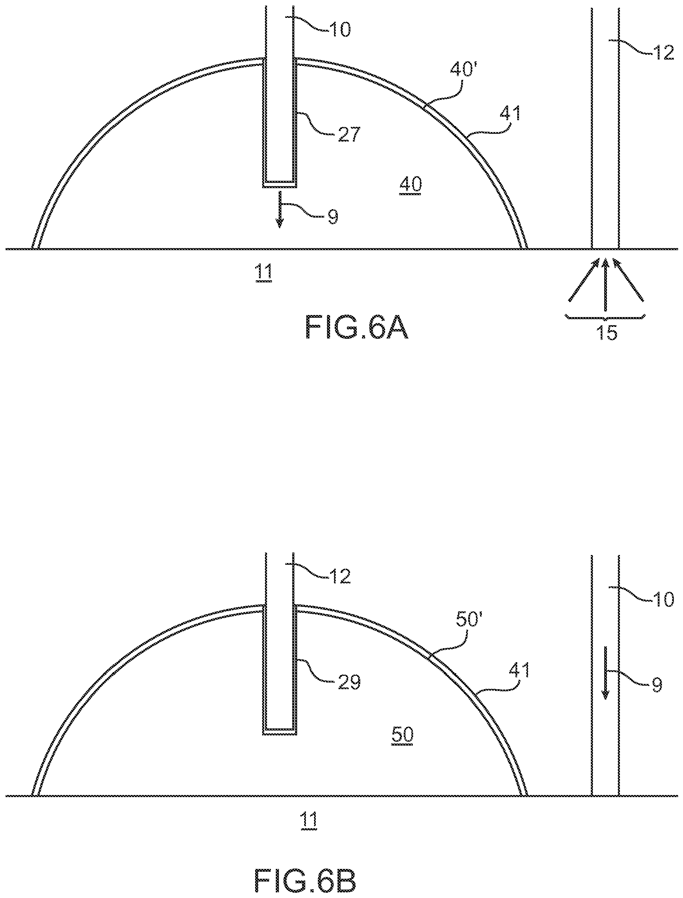

Il est possible dans un mode comparatif (figure 6A) d'avoir une matrice 40 comportant une seule cavité 27, par exemple pour une fibre 10 d'excitation. La fibre de collection 12 est alors positionnée en dehors de la matrice, pour pouvoir capter un signal 15 de fluorescence ou de diffusion en provenance du milieu 11 En variante, une matrice 50 (figure 6B) comporte une seule cavité 29 pour une fibre 12 de collection du signal. La fibre d'excitation 12 est alors positionnée en dehors de la matrice, pour amener un signal 9 d'excitation, ou bien il n'y a pas de fibre d'excitation et le rayonnement lumineux incident est amené directement de la source, située à distance de la matrice 50. On pourra de préférence faire en sorte que tout ou partie rayonnement lumineux produit par la source traverse une partie de la matrice 50, ce qui permet de conserver l'effet technique de diffusion du signal lumineux incident dans la matrice 50 avant d'atteindre le milieu 11 à étudier. Autrement dit, la source lumineuse peut-être une source Laser ou une fibre optique délivrant un faisceau lumineux dirigé vers le milieu 11 et indicent à la surface extérieure 50' de la matrice 50. Ce mode permet de tirer profit de la diffusion du signal d'excitation dans la matrice 50, comme on l'a vu précédemment.

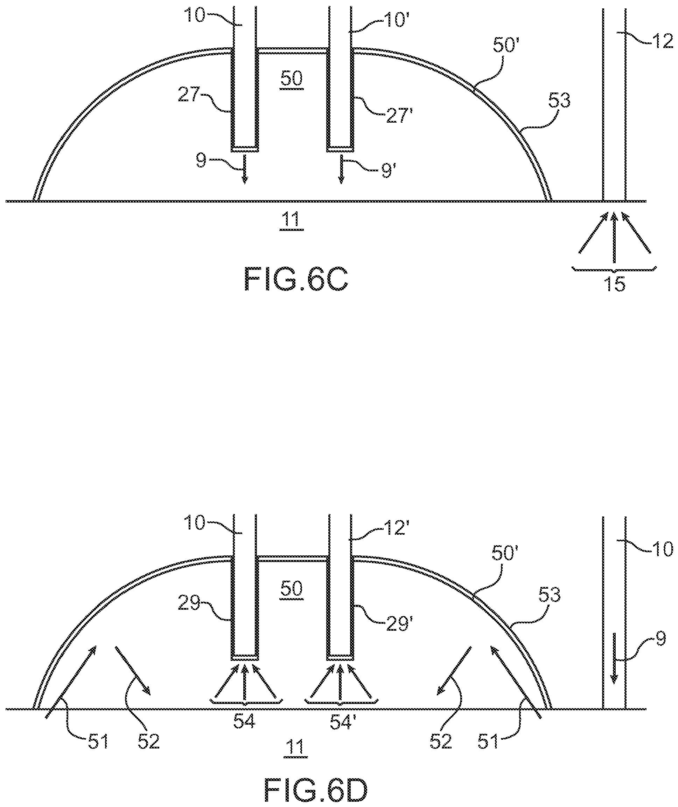

La profondeur de chaque cavité 27, 29 est de préférence adaptée à la nature de la fibre qu'elle reçoit, en fonction des considérations ci dessus. Une matrice 50 avec deux (ou même plus de deux) fibres 10, 10' d'excitation est aussi possible dans ce mode comparatif (figure 6C), tandis que là encore une fibre de collection 12 est positionnée en dehors de la matrice. Dans ce cas, la fibre de collection peut-être remplacée par un détecteur, distant de la surface du milieu 11, mais optiquement couplé à cette surface de façon à collecter une partie du signal lumineux émergeant de cette surface. De même une matrice 50 avec deux (ou même plus de deux) fibres 12, 12' de collection possible dans ce mode comparatif (figure 6D), tandis qu'une ou plusieurs fibre d'excitation 10 est positionnée en dehors de la matrice.

Lors de l'utilisation du dispositif qui a été décrit ci-dessus on applique la surface 22 de la matrice ou des matrices contre l'échantillon à analyser. On introduit la ou les fibres dans la ou les matrices correspondantes, par exemple avant de positionner celle(s) - ci contre l'échantillon.

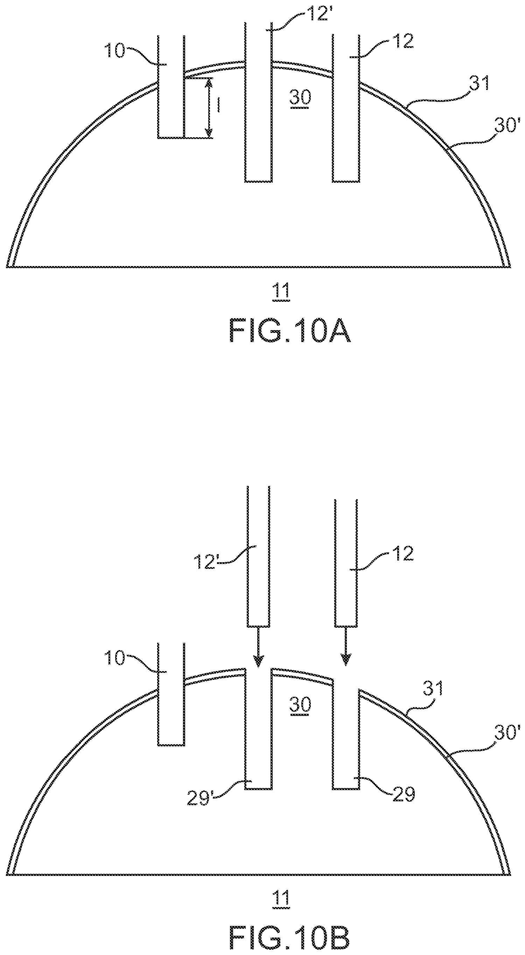

Selon un autre mode de réalisation de l'invention, dont un exemple est représenté en figure 10A, la ou les fibres est/sont intégrée(s) dans la matrice lors de la fabrication de cette dernière, par exemple par moulage. Ceci permet un meilleur couplage optique entre l'extrémité de chaque fibre et le matériau de la matrice. Par conséquent, dans ce mode de réalisation, on ne prépare pas, lors de la fabrication de la matrice, un ou des logements tels que les logements 27, 29 de la figure 2 : l'extrémité d'au moins une fibre optique est placée de manière permanente dans le matériau de la matrice, par exemple sur une longueur l (distance entre la surface 30' de la masse diffusante, non destinée à être appliquée contre le milieu étudié 11, et l'extrémité de la fibre optique disposée dans cette masse) de quelques millimètres à quelques centimètres, par exemple comprise entre 1 mm, ou 5 mm, ou 10 mm, et 1 cm, ou 5 cm, ou 10 cm. On peut, pour obtenir un tel dispositif, réaliser un moulage de la matière de la matrice autour d'une ou plusieurs fibres optiques. De la même manière, on peut réaliser une ou plusieurs fibres intégrée(s) dans une matrice qui a la forme de l'une quelconque de celles qui ont été décrites ci-dessus en liaison avec les figures 2 - 6D.

Dans ce mode de réalisation, les extrémités des fibres sont également placées, mais de manière permanente, à des distances H et h qui peuvent avoir les caractéristiques et/ou les valeurs qui ont été expliquées ci-dessus. Les avantages, en termes optiques, sont les mêmes que ceux qui ont été décrits ci-dessus dans le cas de matrices présentant des logements dans lesquels les fibres sont introduites. En particulier, l'effet élargissement du faisceau incident est le même. En outre, on a le même effet avantageux, dans le cas d'une fibre de collection 12 placée en permanence dans une matrice telle que la matrice 30, que celui qui a été décrit ci-dessus, en liaison avec la figure 3, avec l'exemple des faisceaux 51,52,54. La forme et/ou les caractéristiques du matériau de la matrice, et notamment sa nature, et/ou son coefficient de diffusion réduit, et/ou son coefficient d'absorption, est/sont de préférence choisie(s) parmi celles qui ont déjà été indiquées ci-dessus. Il en va de même pour le rayonnement incident utilisé, et la gamme de longueur d'onde préférentielle dans laquelle elle peut être choisie, notamment l'infrarouge ou le proche infrarouge. Un dispositif selon ce mode de réalisation est donc « prêt à l'emploi », c'est-à-dire que l'on n'a pas besoin, lorsque la matrice est positionnée contre le milieu 11 étudié, de réaliser en outre une opération d'introduction des fibres dans le ou les logements prévus à cet effet dans la matrice.

Suivant une variante de cet autre mode de réalisation, un dispositif selon l'invention comporte une masse diffusante, qui entoure de manière permanente l'extrémité d'une ou plusieurs fibres optiques, et comporte par ailleurs un logement pour l'introduction d'une ou plusieurs autres fibres optiques. Ainsi, on a représenté en figure 10B un mode de réalisation dans lequel une fibre 10 est placée de manière permanente dans la masse 30 du matériau diffusant, tandis que deux fibres de collection 12, 12' sont à introduire dans des logements correspondants 29, 29' Dans la suite, les explications qui seront données en liaison avec l'un des modes de réalisation, dans lequel on introduit une ou plusieurs fibres dans un ou plusieurs logements d'une matrice, s'appliquent également à l'autre mode de réalisation, dans lequel une ou plusieurs fibres sont incorporées ou intégrées dans la matrice lors de la fabrication de cette dernière, à moins qu'il ne soit précisé le contraire.

Dans un mode de réalisation préféré de l'invention mis en œuvre dans une analyse optique, l'énergie lumineuse incidente est amenée vers le milieu 11 examiné, par exemple par une fibre 10. L'énergie est collectée après propagation du rayonnement à analyser dans ce milieu par les moyens 4, 13 de mesure prévus, par exemple un photomultiplicateur ou une caméra telle qu'une caméra CCD, ou CMOS, ou une barrette de CCD, ou une ou plusieurs photodiodes à avalanches, cette énergie étant amenée au détecteur par exemple par une fibre ou le détecteur étant distant de la surface 22 mais optiquement couplé à cette surface. Le montage mis en œuvre peut être le montage de la figure 1, utilisable en combinaison avec le dispositif de couplage de la présente invention, mais l'invention s'applique également aux cas où la source 8 de rayonnement est remplacée par une source continue en temps ou modulée en amplitude. Dans tous les cas, le laser peut être accordable en longueur d'onde, par exemple de 400 nm à 1300 nm, préférentiellement 600 à 950 nm pour exciter différents types de fluorophores.

Le dispositif selon l'invention comporte des moyens numériques de traitement des données mesurées, par exemple un ordinateur programmé à cet effet. Un exemple de procédé d'analyse de signaux d'imagerie de fluorescence résolue en temps est donné dans le document EP-1884765. Ce procédé peut être utilisé pour reconstruire une image à partir d'un tel dispositif.

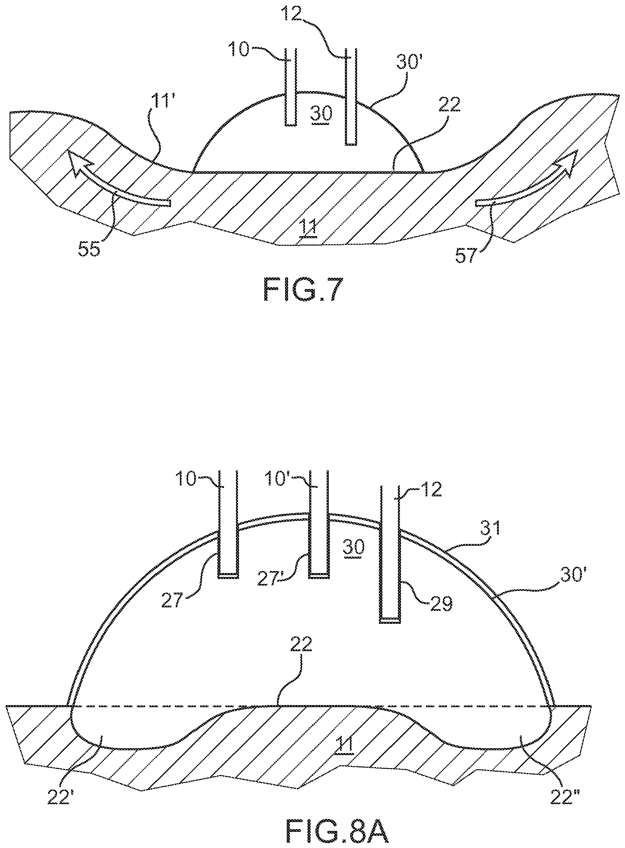

Selon un autre aspect de l'invention, pour un milieu 11 à analyser présentant une certaine souplesse ou élasticité, un meilleur couplage de l'énergie lumineuse d'excitation est obtenu si on enfonce légèrement la matrice 30 dans ce milieu, comme illustré en figure 7. Sur cette figure l'exemple est pris d'une matrice du type de la figure 3, mais le même résultat vaut pour toute autre matrice selon la présente invention.

Dans le cas de tissus vivants, ceci est attribué au fait que le sang, responsable de l'absorption, est alors localement chassé par la pression exercée sur la matrice, comme indiqué par les flèches 55, 57 de la figure 7. L'air (entre la surface 22 de la matrice 30 et la surface 11' du tissu 11) est également chassé, ce qui permet aussi un meilleur couplage en minimisant l'interface tissu/air. La pression exercée sur la matrice 30 permet donc un meilleur couplage du rayonnement incident avec le milieu et un meilleur couplage du signal émis par le milieu avec le dispositif de collection selon l'invention.

Plus généralement, si le milieu étudié contient un fluide, une pression appliquée au dispositif de couplage selon l'invention permet de faire refluer ou d'évacuer ce fluide en dehors des zones étudiées, ce qui contribue à un meilleur signal mesuré.

Afin encore d'assurer un bon couplage entre un dispositif de couplage selon l'invention et le milieu étudié, on peut réaliser une surface 22 d'appui présentant une ou des excroissances ou protubérances 22', 22" pouvant avoir une forme sensiblement arrondie ou convexe, non anguleuse comme illustré en figure 8A.

Le dispositif selon l'invention comporte alors une surface 22 de contact avec le milieu 11 à examiner, surface qui a au moins un rayon de courbure non nul dans un plan perpendiculaire à la surface de ce milieu.

Une telle excroissance ou protubérance est une portion ou une zone de matière qui s'étend au-delà de la surface principale d'appui 22, en direction de la matière à analyser. Tandis que la surface principale d'appui 22 est en contact avec la surface 11 de la matière à analyser, la protubérance entre plus profondément dans cette matière. On forme ainsi une zone de matière de la matrice 20, zone qui va être entourée de matière à analyser, comme on le comprend d'après la figure 8A qui représente la matrice avec deux protubérances 22', 22'' enfoncées dans la matière 11.

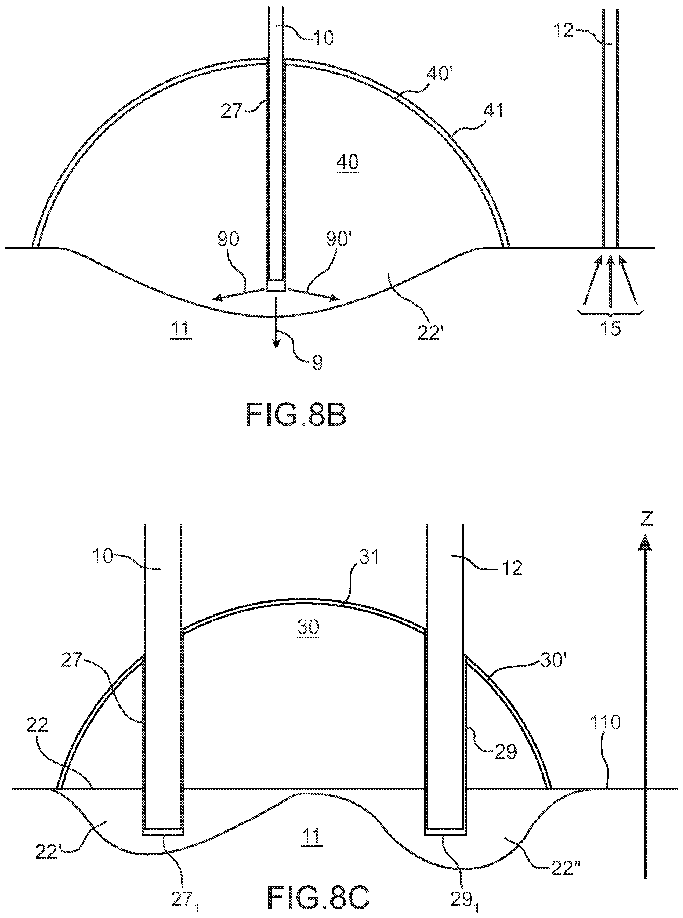

La figure 8B représente une variante qui sert comme mode comparatif. Cette fois on prend l'exemple d'un système du type de la figure 6A, avec une fibre d'excitation dans la matrice. L'extrémité de la fibre 10 est placée dans une protubérance 22', la pénétration des photons dans le milieu à analyser est donc beaucoup plus efficace, puisque des photons ou du rayonnement 90, 90' qui auraient pu s'échapper, par exemple latéralement, sans donner lieu à une interaction avec la matière 11, vont au contraire rencontrer celle-ci et donc renforcer et rendre plus efficace le signal d'excitation.

Comme illustré en figure 8D, l'extrémité de la fibre d'excitation 10 est placée au centre de la courbure de la protubérance 22', cette dernière ayant un rayon de courbure sensiblement constant. Cette disposition permet d'obtenir une excitation homogène sur toute la surface du tissu en contact avec la protubérance.

Dans cet exemple comparatif, la fibre de collection du signal est située à côté de la matrice 40.

La collection du signal est elle aussi renforcée si la fibre 12 est également placée dans la matrice (comme en figure 3) et si son extrémité est aussi placée dans une protubérance 22'' : c'est la structure de la figure 8C. Sur cette figure on voit, comme sur la figure 8B, que l'extrémité 27₁, 27₂ de chaque cavité 27, 29 est disposée, suivant l'axe z, sous la surface 110 qui délimite, de part et d'autre du dispositif, le milieu 11 à examiner.

Dans tous les cas les excroissances 22', 22" peuvent être en matériau identique au matériau dont est constituée la matrice 20.

Dans tous les cas, on peut ajouter, entre la surface 22 et la surface de la matière à examiner, un liquide ayant préférentiellement un indice de réfraction voisin du milieu ou du matériau. (Par exemple visqueux, par exemple de l'intralipide pour que le couplage soit encore meilleur. Ce liquide est préférentiellement transparent et à diffusion négligeable. Sans ce liquide le fait d'appliquer une pression pour chasser l'air entre la surface 22 de la matrice 20 et la surface 110 du matériau 11 à examiner permet déjà d'obtenir un bon couplage.

## Revendications

1. Dispositif d'imagerie optique diffuse d'un milieu (11), comportant :
a) des moyens (8) formant source de rayonnement pour former un rayonnement incident sur le milieu (11), à au moins une première longueur d'onde,
b) des moyens de détection (4, 13) d'un signal diffusé ou de fluorescence, provenant du milieu étudié, et des moyens numériques de traitement des données mesurées avec ces moyens de détection pour reconstruire une image du milieu (11)
c) un dispositif de couplage comportant :
- une masse (20, 30, 40, 50), comportant une surface (22) d'appui, à appliquer contre la surface (110) dudit milieu (11) à examiner, ladite masse étant en matériau diffusant pour une longueur d'onde guidée par l'une au moins des fibres optiques, ce matériau diffusant ayant un coefficient de diffusion réduit supérieur à 0,1 cm⁻¹ et inférieur à 700 cm⁻¹,
- au moins une fibre optique (12, 12') de collection pour prélever un signal en provenance dudit milieu (11), et l'amener aux moyens de détection et au moins une fibre optique d'excitation, pour amener le rayonnement incident sur le milieu à examiner, chaque fibre optique ayant une extrémité disposée de manière permanente dans le matériau diffusant, à distance d'une surface, de la masse du dispositif de couplage, non destinée à être appliquée contre la surface (110) du milieu (11) à examiner, comprise entre 1 mm et 10 cm, ou bien le dispositif de couplage comportant au moins un logement apte à recevoir ladite une fibre optique, ce logement étant réalisé et se prolongeant dans la masse de matériau diffusant,
- l'extrémité de la fibre optique de collection ou le fond du logement apte à recevoir la fibre optique de collection étant situé(e) à une distance (h) de la surface d'appui (22) inférieure à une distance (H), de la surface d'appui (22), à laquelle est situé(e) l'extrémité de la fibre optique d'excitation ou le fond du logement apte à recevoir la fibre optique d'excitation,
**caractérisé en ce que** :
- du matériau diffusant est disposé entre la surface d'appui (22) et l'extrémité de la fibre optique de collection ou le fond du logement apte à recevoir la fibre optique de collection, afin de ramener vers l'extrémité de la fibre optique (12, 12') de collection des photons provenant du milieu étudié (11) et qui ne sont pas dirigés directement vers l'extrémité de cette fibre optique, en permettant à ces photons d'être diffusés dans la masse en matériau diffusant.

2. Dispositif selon la revendication 1, le matériau diffusant ayant un coefficient d'absorption supérieur à 0,01 cm⁻¹ et inférieur à 1 cm⁻¹.

3. Dispositif selon l'une des revendications 1 ou 2, comportant en outre une couche absorbante (31) recouvrant au moins partiellement la masse (20, 30, 40, 50) en matériau diffusant.

4. Dispositif selon l'une des revendications 1 à 3, la tache (37) d'au moins un faisceau incident, sur la surface d'appui (22), destinée à être en contact avec le milieu (11) à examiner, ayant une surface Sₜ comprise entre 1 mm² et quelques dizaines de cm².

5. Dispositif selon l'une des revendications 1 à 4, la surface d'appui (22) comportant en outre une ou plusieurs protubérances (22', 22'') convexes et non anguleuse.

6. Dispositif selon la revendication 5, comportant au moins un logement (27, 27', 29, 29') dont le fond est situé sensiblement dans, ou au niveau de, la protubérance, et/ou comportant au moins une fibre dont l'extrémité est disposée dans, ou au niveau de, ladite protubérance.

7. Dispositif selon l'une des revendications 1 à 6, ladite source (8) de rayonnement étant une source en impulsions, ou continue en temps ou modulée en amplitude.

8. Procédé d'imagerie optique diffuse d'une partie d'un milieu (11), dans lequel on utilise au moins un dispositif selon l'une des revendications 1 à 7, et dans lequel :
- on applique contre ce milieu le dispositif de couplage,
- on réalise un éclairement ou une excitation optique du milieu (11) à l'aide des moyens (8) formant source de rayonnement pour former un rayonnement incident sur le milieu (11), puis pour introduire ce rayonnement incident dans le milieu (11).
- on prélève un signal optique en provenance du milieu (11), à l'aide de la fibre optique de collection ou d'au moins une fibre optique de collection (12, 12').

9. Procédé selon la revendication 8, dans lequel on applique un fluide entre la surface d'appui (22) du milieu diffusant du dispositif de couplage et la surface (110) du milieu (11) à examiner.

10. Procédé selon l'une des revendications 8 ou 9, dans lequel on applique une pression au dispositif de couplage, lorsqu'il est en position contre le milieu (11), de manière à faire refluer un fluide contenu par ce milieu hors d'une partie de ce milieu, vers une zone située à la périphérie du dispositif de couplage.

## Patentansprüche

1. Vorrichtung zur diffusen optischen Abbildung eines Mediums (11), enthaltend:
a) eine Strahlungsquelle bildende Mittel (8) zum Bilden einer in das Medium (11) einfallenden Strahlung mit zumindest einer ersten Wellenlänge,
b) Mittel (4, 13) zum Erfassen eines gestreuten oder fluoreszierenden Signals von dem untersuchten Medium und digitale Mittel zum Verarbeiten der mit diesen Erfassungsmitteln gemessenen Daten, um ein Bild des Mediums (11) zu rekonstruieren,
c) eine Kopplungsvorrichtung, enthaltend:
- eine Masse (20, 30, 40, 50) mit einer Abstützfläche (22), die an die Oberfläche (110) des zu untersuchenden Mediums (11) anzulegen ist, wobei die Masse aus diffundierendem Material bei einer Wellenlänge besteht, die von zumindest einer der Lichtleitfasern geführt wird, wobei dieses diffundierende Material einen reduzierten Diffusionskoeffizienten von über 0,1 cm⁻¹ und unter 700 cm⁻¹ aufweist,
- zumindest eine Sammel-Lichtleitfaser (12, 12') zum Abgreifen eines Signals von dem Medium (11) und zum Zuführen desselben zu den Erfassungsmitteln, sowie zumindest eine Anregungs-Lichtleitfaser zum Zuführen der in das zu untersuchende Medium einfallenden Strahlung, wobei jede Lichtleitfaser ein Ende aufweist, das dauerhaft in dem diffundierenden Material in einem Abstand zwischen 1 mm und 10 cm von einer Oberfläche der Masse der Kopplungsvorrichtung angeordnet ist, die nicht dazu bestimmt ist, an die Oberfläche (110) des zu untersuchenden Mediums (11) angelegt zu werden, oder wobei die Kopplungsvorrichtung zumindest eine Aufnahme umfasst, die zum Aufnehmen der einen Lichtleitfaser geeignet ist, wobei diese Aufnahme in der Masse des diffundierenden Materials ausgebildet ist und sich darin fortsetzt,
- wobei das Ende der Sammel-Lichtleitfaser bzw. der Grund der zum Aufnehmen der Sammel-Lichtleitfaser geeigneten Aufnahme in einem Abstand (h) von der Abstützfläche (22) liegt, der kleiner als ein Abstand (H) von der Abstützfläche (22) ist, an dem sich das Ende der Anregungs-Lichtleitfaser bzw. der Grund der zum Aufnehmen der Anregungs-Lichtleitfaser geeigneten Aufnahme befindet,
**dadurch gekennzeichnet, dass**
- diffundierendes Material zwischen der Abstützfläche (22) und dem Ende der Sammel-Lichtleitfaser bzw. dem Grund der zum Aufnehmen der Sammel-Lichtleitfaser geeigneten Aufnahme angeordnet ist, um an das Ende der Sammel-Lichtleitfaser (12, 12') Photonen zurückzuführen, die von dem zu untersuchenden Medium (11) stammen und nicht direkt zum Ende dieser Lichtleitfaser gerichtet sind, indem es diesen Photonen ermöglicht wird, gestreut zu werden.

2. Vorrichtung nach Anspruch 1, wobei das diffundierende Material einen Absorptionskoeffizienten von über 0,01 cm⁻¹ und unter 1 cm⁻¹ aufweist.

3. Vorrichtung nach einem der Ansprüche 1 oder 2, ferner enthaltend eine absorbierende Schicht (31), welche zumindest teilweise die Masse (20, 30, 40, 50) aus diffundierendem Material überdeckt.

4. Vorrichtung nach einem der Ansprüche 1 bis 3, wobei der Fleck (37) zumindest eines einfallenden Strahls auf der Abstützfläche (22), die dazu bestimmt ist, mit dem zu untersuchenden Medium (11) in Kontakt zu gelangen, eine Fläche Sₜ zwischen 1 mm² und mehreren zehn cm² aufweist.

5. Vorrichtung nach einem der Ansprüche 1 bis 4, wobei die Abstützfläche (22) ferner einen oder mehrere konvexe, nicht eckige Vorsprünge (22', 22") aufweist.

6. Vorrichtung nach Anspruch 5, enthaltend zumindest eine Aufnahme (27, 27', 29, 29'), deren Grund im Wesentlichen in bzw. an dem Vorsprung liegt, und/oder enthaltend zumindest eine Faser, deren Ende in bzw. an dem Vorsprung angeordnet ist.

7. Vorrichtung nach einem der Ansprüche 1 bis 6, wobei die Strahlungsquelle (8) eine Impulsquelle oder eine zeitkontinuierliche oder amplitudenmodulierte Quelle ist.

8. Verfahren zur diffusen optischen Abbildung eines Teils eines Mediums (11), wobei zumindest eine Vorrichtung nach einem der Ansprüche 1 bis 7 verwendet wird, und wobei
- an dieses Medium die Kopplungsvorrichtung angelegt wird,
- eine Ausleuchtung bzw. optische Anregung des Mediums (11) mit Hilfe der eine Strahlungsquelle bildenden Mittel (8) erfolgt, um eine Strahlung zu bilden, die in das Medium (11) einfällt, und um diese einfallende Strahlung in das Medium (11) einzubringen,
- ein optisches Signal von dem Medium (11) mit Hilfe der Sammel-Lichtleitfaser bzw. zumindest einer Sammel-Lichtleitfaser (12, 12') abgegriffen wird.

9. Verfahren nach Anspruch 8, wobei ein Fluid zwischen der Abstützfläche (22) des diffundierenden Mediums der Kopplungsvorrichtung und der Oberfläche (110) des zu untersuchenden Mediums (11) eingebracht wird.

10. Verfahren nach einem der Ansprüche 8 oder 9, wobei die Kopplungsvorrichtung mit Druck beaufschlagt wird, wenn sie sich in Stellung am Medium (11) befindet, so dass ein im Medium enthaltenes Fluid aus einem Teil dieses Mediums in einen Bereich am Umfang der Kopplungsvorrichtung zurückfließt.

## Claims

1. Diffuse optical imaging device to image a medium (11), comprising:
a) means (8) forming a radiation source to form incident radiation on the medium (11), at least at a first wavelength,
b) means (4, 13) to detect a diffused or fluorescence signal, emanating from the examined medium, and digital means to process data measured with said means to detect data reconstruct an image from of said medium (11),
c) a coupling device comprising:
- a mass (20, 30, 40, 50) comprising a bearing surface (22) to be applied against the surface (110) of the medium (11) to be examined, said mass being in a diffusing material for a wavelength guided by at least one of the optical fibres, this material having a reduced diffusion coefficient higher than 0.1 cm⁻¹ and less than 700 cm⁻¹;
- at least one collection optical fibre (12, 12') to collect a signal emanating from a medium (11), and to convey it towards the detection means, and at least one excitation optical fibre to bring the incident radiation onto the medium to be examined, each optical fibre having one end arranged permanently in said mass to be applied against the surface (110) of the medium (11) to be examined, at a distance of a surface of the mass of the coupling device, not intended to be applied against the surface (110) of the examined medium (11), comprised between 1 mm and 1 cm, or the coupling device comprising at least one housing capable of receiving said optical fibre, this housing being provided and extending in said mass of diffusing material,
- the end of the collection optical fibre or the bottom of the housing capable of receiving the collection optical fibre being located at a distance (h) from the bearing surface (22) shorter than the distance (H) from the bearing surface (22) at which the end of the excitation optical fibre or the bottom of the housing capable of receiving the excitation optical fibre is located,
**characterized in that**
- diffusing material is arranged between the bearing surface (22) and the end of the collecting fibre or the bottom of the housing capable of receiving the collection optical fibre, in order to bring back towards the end of the collection optical fibre (12, 12') photons derived from the examined medium (11) and which are not directly directed towards the end of this optical fibre, by allowing these photons to be diffused in the mass of diffusing material.

2. Device according to claim 1, the diffusing material having an absorption coefficient higher than 0.01 cm⁻¹ and less than 1 cm⁻¹.

3. Device according to one of claims 1 or 2, further comprising an absorbent layer (31) at least partly covering the mass (20, 30, 40, 50) in diffusing material.

4. Device according to any of claims 1 to 3, the spot (37) of at least one incident beam, on the bearing surface (22) intended to be in contact with the medium (11) to be examined, having a surface area Sₜ of between 1 mm² and a few tens of cm².

5. Device according to any of claims 1 to 4, the bearing surface (22) further comprising one or more convex protuberances (22', 22") with no sharp edges.

6. Device according to claim 5, comprising at least one housing (27, 27', 29, 29') whose bottom is located substantially in or at the protuberance, and/or comprising at least one fibre whose end is arranged in or at said protuberance.

7. Device according to any of claims 1 to 6, said radiation source (8) being a pulsed, or continuous-in time or amplitude-modulated source.

8. Examination method of optical tomography type to examine part of a medium (11), wherein at least one device is used according to any of claims 1 to 7, and wherein:
- the coupling device is applied against this medium,
- the medium (11) is illuminated or subjected to optical excitation using means (8) forming a radiation source to form incident radiation on the medium (11), then to cause this incident radiation to enter the medium (11).
- an optical signal emanating from the medium (11) is collected by means of the collection optical fibre or of at least one collection optical fibre (12, 12').

9. Method according to claim 8, wherein a fluid is applied between the bearing surface (22) of the diffusing medium of the coupling device and the surface (110) of the medium (11) to be examined.

10. Method according to either of claims 8 or 9, wherein a pressure is applied to the coupling device when it is in position against the medium (11), to cause a fluid contained in this medium to flow out of part of this medium towards a region located on the periphery of the coupling device.
